# NEUE EUROPÄISCHE PATENTSCHRIFT

(11) **EP 1 479 411 B2**
(45) Veröffentlichungstag und Bekanntmachung des Hinweises auf die Entscheidung über den Einspruch: **18.01.2012**
(45) Hinweis auf die Patenterteilung: 13.04.2005
(21) Anmeldenummer: 03011556.2
(22) Anmeldetag: 21.05.2003
(51) Int. Cl.: A61N 5/10

(54) **Vorrichtung zur überwachten Tumorbestrahlung**
Apparatus for monitored tumour irradiation
Dispostif d'irradiation de tumeur controlée

(43) Veröffentlichungstag der Anmeldung: 24.11.2004
(73) Patentinhaber: Varian Medical Systems Particle Therapy GmbH, 51429 Bergisch Gladbach (DE)
(72) Erfinder: Rinecker, Hans, PD Dr. med. Dr. med. habil., 81379 München (DE); Hauffe, Jörg, Dr., 81541 München (DE)
(74) Vertreter: Bergmann, Michael

(56) Entgegenhaltungen:
- EP-A- 1 419 800
- EP-A1- 1 419 801
- WO-A-03/018133
- WO-A1-01/00276
- WO-A1-03/018132
- DE-A- 10 051 370
- US-A- 4 885 998
- US-A- 4 924 781
- US-A- 5 039 057
- US-A- 5 117 829
- US-A- 5 207 223
- US-A- 5 825 845
- US-A- 6 094 760
- US-A- 6 118 848
- US-B1- 6 307 914

## Beschreibung

Die vorliegende Erfindung betrifft eine Vorrichtung zur überwachten Tumorbestrahlung gemäß dem Oberbegriff des Anspruchs 1.

Teilchenstrahlentherapie wird hauptsächlich verwendet, um lokalisierte Formen von Krebs zu behandeln. Üblicherweise wird ein atomares Partikel, d.h. ein Elektron, Proton, Neutron oder Schwerion, oder Röntgenstrahlung aus einer Strahlabgabevorrichtung auf einen speziellen Zielbereich im Patienten emittiert, der auch als Targetvolumen bezeichnet wird. Die Partikel geben Energie an die Körperzellen innerhalb des Zielbereichs ab, wodurch diese aufgrund von Radikalbildung in der Zellflüssigkeit zerstört werden.

Eine Bestrahlungsform, die sich in den letzten Jahren als besonders effizient herausgestellt hat, ist die Bestrahlung mit Protonen, welche die meiste Energie in einem sogenannten Bragg-Peak unmittelbar vor ihrem endgültigen Abstoppen abgeben, so daß auch tieferliegende Tumore bestrahlt werden können, ohne das umliegende Gewebe stark zu schädigen. Durch Auswahl der Energie des Protonenstrahls können die Protonen derart in den Körper geleitet werden, daß sie genau im Targetvolumen des Tumors zum Stillstand kommen und dort den Hauptteil ihrer Energie an die kranken Zellen abgeben. Eine bereits in Betrieb befindliche Protonentherapieanlage ist im Loma Linda University Medical Center integriert und detaillierter in der US 4,870,287 beschrieben.

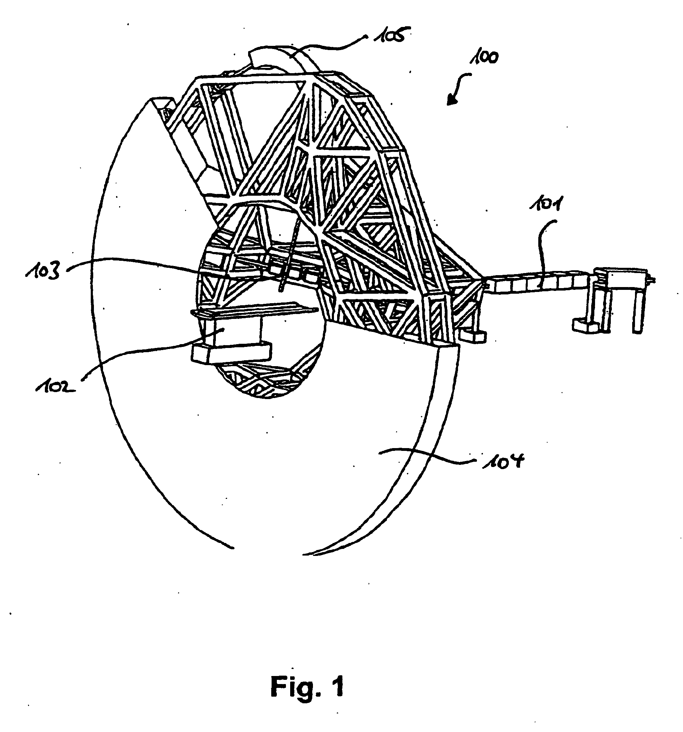

Für Bestrahlungen von Tumoren innerhalb des menschlichen Körpers werden sogenannte Gantries, eines dargestellt in Fig. 1, verwendet. Derartige Gantries 100 sind große, drehbar gelagerte Kostruktionen in Form eines Ringes bzw. Hohlzylinders, die zur Strahlführung eines über eine Strahlzuführungseinheit 101 eingespeisten Teilchenstrahls dienen und in deren Innenraum eine Patientenpositionierungsvorrichtung 102 angeordnet ist, auf der der Patient bestrahlt wird. Die Protonenstrahlabgabevorrichtung 103 kann mit dem Ring des Gantry 104 um 360° um den Patienten gedreht werden, ohne daß eine Beeinträchtigung des Protonenstrahls auftritt, der in der Strahlführung 105 läuft. Dadurch kann der Patient von verschiedenen Seiten bestrahlt werden, was eine genaue Abtastung der dreidimensionalen Form des Tumorgewebes ermöglicht und das umliegende Gewebe noch mehr schont.

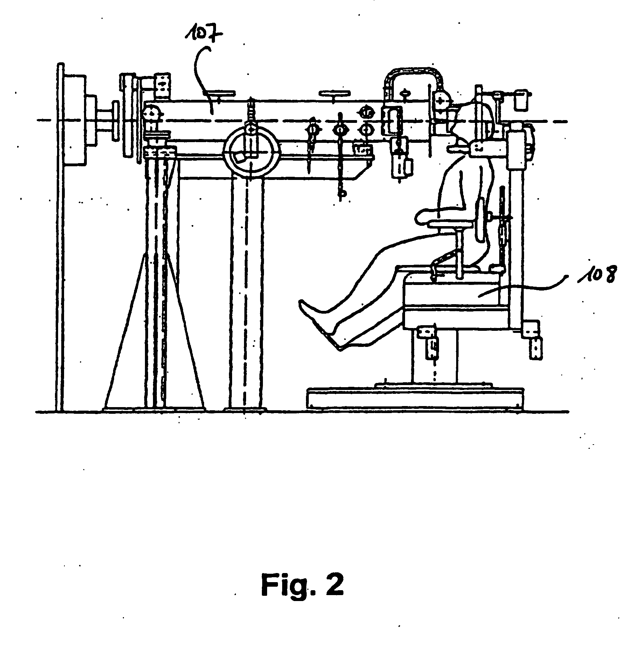

Für Augenbestrahlungen können ortsfeste Protonenstrahlabgabevorrichtungen (siehe Fig. 2) verwendet werden, die ebenfalls eine Strahlabgabevorrichtung 107 und eine Patientenpositionierungsvorrichtung 108, meist einen Sitz, aufweisen.

Wie erwähnt liefert Protonentherapie einige signifikante klinische Vorteile gegenüber anderen Arten von Therapien, allerdings ist es äußerst wichtig, daß der Patient im Verhältnis zur Protonenstrahlabgabevorrichtung sehr genau positioniert wird, so daß das Targetvolumen optimal getroffen wird. Bei falscher Positionierung würde der Protonenstrahl die meiste Energie an gesunde Zellen innerhalb des Körpers des Patienten abgeben und diese zerstören. Um dies zu verhindern, wird im Vorfeld die Position des Targetvolumens im Hinblick auf einen oder mehrere Fixpunkte innerhalb des Körpers des Patienten bestimmt. Hierzu werden die Patienten auf einer speziell angefertigten Patientenpositionierungsvorrichtung fixiert. Dies kann entweder eine an die Körperformen angepaßte Liege mit entsprechenden Vertiefungen sein, oder ein speziell einzustellender Behandlungsstuhl bei der Augenbestrahlung. Die Fixpunkte werden üblicherweise an der Skelettstruktur des Patienten gewählt, bei beweglichen Körperteilen wie z.B. Augen können auch künstliche Fixpunkte, etwa Metallclips, in die Sklera des Auges eingesetzt werden und als Referenzsystem dienen.

Die Position des Targetvolumens wird dann beispielsweise durch eine Kombination von üblichen Kernspin- und CT-Aufnahmen bestimmt und im oben beschriebenen Referenzsystem lokalisiert. Unter Verwendung der Daten des CT-Scans wird auch ein Behandlungsplan für den Patienten erstellt (siehe z.B. US 5,117,829). Der Behandlungsplan wird über eine Computersimulation berechnet, insbesondere die Anzahl von Bestrahlungen und die Winkel, unter denen die Strahlen am besten die Krebszellen erreichen.

Üblicherweise erhält der Patient schließlich über mehrere Tage hinweg periodische Behandlungen, bei denen das Targetvolumen wiederholt mit einem Protonenstrahl bestrahlt wird. Um sicher zu stellen, daß der Patient bei jeder Behandlung richtig positioniert ist, werden die Patienten wieder auf derselben Patientenpositionierungsvorrichtung fixiert. Um jede Abweichung vom Sollzustand auszuschließen und Positionskorrekturen vornehmen zu können, sollte die Bestrahlungseinheit in der Lage sein, die Referenzpunkte im menschlichen Körper, unter Bezugnahme auf die man den Tumor zuvor lokalisiert hatte, wieder zu bestimmen. An diesen Punkten läßt sich erkennen, ob sich der Patient in der richtigen Position befindet, für die der Bestrahlungsplan entwickelt wurde. Hierfür sind bislang mehrere Verfahren vorgeschlagen worden.

Zum einen wurde vorgeschlagen, mittels einer üblichen Röntgenanordnung ein Bild des sich in der Patientenpositionierungsvorrichtung befindlichen Patienten aufzunehmen, um die Lage der Fixpunkte mit der gewünschten Lage zu vergleichen. Ein derartiges System ist beispielsweise aus der US 5,825,845 bekannt. Die Röntgenquelle ist hierbei im Bereich der Protonenstrahlabgabevorrichtung angeordnet und kann in den Protonenstrahlengang geschoben werden, so daß die Röntgenstrahlung aus der selben Richtung wie die Protonenstrahlung auf den Patienten trifft. Auf der gegenüberliegenden Seite der Patientenpositionierungsvorrichtung ist ein Röntgendetektor angeordnet, der die Röntgenstrahlung aufnimmt. Mit dieser Anordnung kann z.B. vor der Behandlung ein Röntgenbild aufgenommen werden, und bei Nichtübereinstimmen der Fixpunkte kann der Patient leicht bewegt werden bzw. die im Gantry vorhandene Einstellmechanik leicht derart justiert werden, daß die Lage des Patienten völlig identisch ist zu der Lage, in der die Aufnahmen gemacht wurden, welche zur Bestimmung des Bestrahlungsplans gedient hatten. Eine derartige Vorrichtung besitzt jedoch den großen Nachteil, daß lediglich aus einer Richtung Röntgenbilder aufgenommen werden können, so daß bei ungünstiger Lage des Tumors die knöcherne Skelettstruktur und damit die Fixpunkte oft nicht deutlich sichtbar sind. Außerdem ist die Mechanik, die für das Einbringen der beweglichen Röntgenquelle in den Protonenstrahlengang verwendet wird, relativ kompliziert und störanfällig. Wenn während der Bestrahlung eine Positionsüberprüfungsaufnahme gemacht werden soll, wird aufgrund der Umbaumaßnahmen die Behandlungszeit enorm erhöht. Schließlich ist aufgrund der räumlichen Anordnung der Röntgenquelle der Luftweg des Partikelstrahls relativ lang, wodurch die Strahlqualität verringert wird.

Eine weitere Form der Patientenpositionskontrolle wird in EP 1 121 957 A2 vorgeschlagen. Hier ist die Bestrahlungseinheit mit einer Kernspineinheit versehen, die annähernd simultan zur Bestrahlung eine nichtinvasive Lokalisierung des Tumors liefert. Dieses Verfahren ist zwar sehr genau, unterliegt aber den räumlichen Beschränkungen, die für eine Kernspinaufnahme nötig sind, so daß z.B. von vornherein eine Bestrahlung in einem Gantry ausgeschlossen ist.

Eine weitere Form der Patientenpositionskontrolle ist aus der WO 00/59575 bekannt, bei der eine Mehrzahl von Positionsdetektoranordungen innerhalb eines Gantrys vorgesehen sind, die jeweils die Entfernung zwischen der vorderen Oberfläche des Positionssensors und einer gegenüberliegenden Oberfläche des Patienten messen. Ein derartiges Verfahren mißt jedoch nur die Oberfläche des Patienten und kann keine Informationen für die relative Positionsbestimmung von Targetvolumen und Referenzsystem liefern.

US 5,207,223 beschreibt eine Vorrichtung zur Bestrahlung von Tumorgewebe im Kopfbereich mittels Photonen. Die Vorrichtung weist eine Therapiestrahlabgabevorrichtung auf, die in einem drehbaren Gantry angeordnet ist, zwei orthogonal zueinander angeordnete Röntgenquellen sowie gegenüberliegende Röntgendetektoren, die zur Positionsverifizierung des Patienten bzw. des zu bestrahlenden Tumorgewebes dienen. Dabei befinden sich die beiden Röntgenquellen seitlich von der Therapiestrahlabgabevorrichtung

US 6,307,914 beschreibt ebenfalls eine Vorrichtung zum Bestrahlen von Tumorpatienten mittels Photonen oder anderen geeigneten Strahlungsarten. Zur Positionsidentifizierung des Patienten bzw. des Tumors sind ebenfalls Röntgenquellen und gegenüberliegende Röntgendetektoren vorgesehen, die nahezu beliebig im Raum angeordnet werden können.

Die WO 03/018133 A1 und die WO 03/018132 A1 zeigen eine Bestrahlungsvorrichtung mittels Photonen, die auch als Gantry-artige Anordnung ausgestaltet sein kann. Hierbei ist erwähnt, dass die zur Positionsverifizierung dienenden Röntgenstrahlendetektoren einen Winkel von zwischen 20° und 60° zur Strahlvorrichtung aufweisen, vorzugsweise zwischen 30° und 45°. Dadurch soll gewährleistet sein, dass sie sich nicht gegenseitig beeinflussen und die gelieferte Abbildung eine ausreichende Genauigkeit besitzt.

Der vorliegenden Erfindung liegt daher die Aufgabe zugrunde, eine Vorrichtung zur überwachten Tumorbestrahlung zu schaffen, die in Gantrys einsetzbar, relativ einfach aufgebaut und wartungsfreundlich ist, und mit der unabhängig von der Strahlungsrichtung, aus der die geladenen Teilchen auf den Patienten gerichtet werden, jederzeit eine exakte und sichere Positionsbestimmung des Patienten möglich ist.

Diese Aufgabe wird durch die Merkmale des Anspruchs 1 gelöst.

Durch die erfindungsgemäße Vorrichtung wird gewährleistet, daß jederzeit und unabhängig von der Strahlrichtung der geladenen Teilchen eine sichere Einschätzung der Patientenposition vorgenommen werden kann. Außerdem kann mit der vorliegenden Erfindung die Konstruktion der Strahlabgabevorrichtung vereinfacht und durch Verkürzung der Luftwege die Qualität des Partikelstrahls verbessert werden.

Außerdem wird eine Bestimmung der Patientenposition von mehreren Seiten gewährleistet, wobei sich die Röntgenstrahlendetektoren in der Zone niedrigster Streustrahlungsintensität der Teilchenstrahlung befinden, so daß Bestrahlung und Positionsverifizierung gleichzeitig durchgeführt werden können.

Vorzugsweise sind die Röntgenstrahlendetektoren im selben Abstand auf beiden Seiten der Strahlabgabevorrichtung angeordnet, was mechanische Vorteile liefert.

Bevorzugt schließen die Röntgenstrahlendetektoren einen Winkel von zwischen 70° und 90° ein. Eine Annäherung an 90° liefert ein breites Informationsspektrum, während ein kleinerer Winkel manchmal konstruktiv einfacher umzusetzen ist.

Es ist mechanisch von Vorteil, daß die Röntgenstrahlendetektoren an der Strahlabgabevorrichtung befestigt sind.

Für eine leichtere Positionskorrektur ist die Vorrichtung zur Patientenpositionierung vorzugsweise drehbar ausgebildet.

Weitere Einzelheiten, Merkmale und Vorteile der vorliegenden Erfindung ergeben sich aus der nachfolgenden Beschreibung unter Bezugnahme auf die Zeichnungen. Darin zeigt:
- Fig. 1: ein Gantrysystem nach dem Stand der Technik,
- Fig. 2: ein Augenbestrahlungssystem nach dem Stand der Technik, und
- Fig. 3: einen Schnitt durch eine erfindungsgemäße Vorrichtung zur überwachten Tumorbestrahlung in einem Gantry.

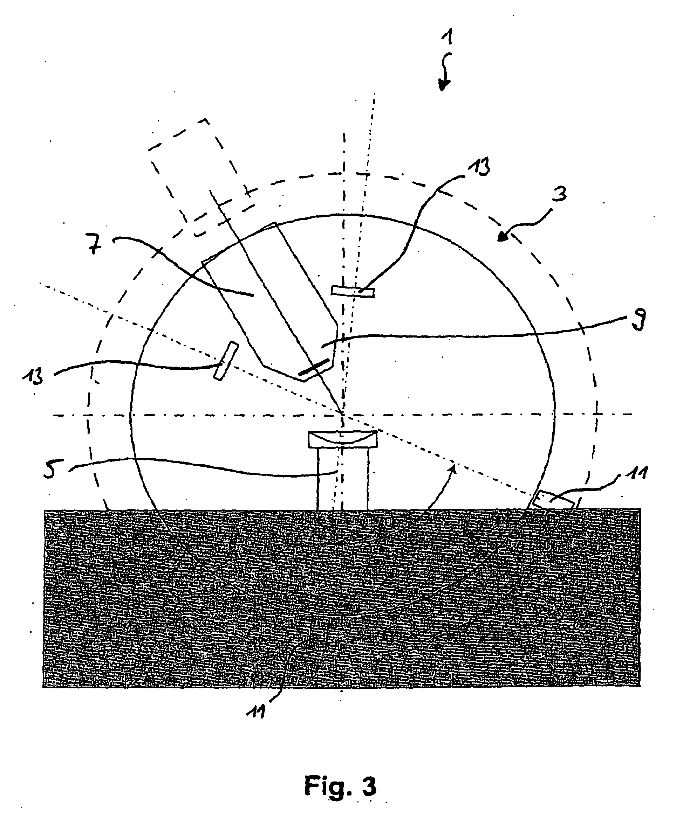

In Fig. 3 ist ein Querschnitt durch eine erfindungsgemäße Vorrichtung zur überwachten Tumorbestrahlung von Krebspatienten gemäß der vorliegenden Erfindung dargestellt. Die Vorrichtung 1 ist innerhalb eines Gantrys 3 angeordnet, wie es bereits aus dem Stand der Technik bekannt ist. Im Bestrahlungsraum befindet sich eine vorzugsweise bewegbare Patentenpositionierungsvorrichtung 5, hier als Liege ausgestaltet, die geeignete Immobilisierungsmittel aufweist, beispielsweise eine an den Patienten angepaßte Mulde in der Liege und weitere aus dem Stand der Technik bekannte Mittel. Im Gantry 3 ist eine Protonenstrahlabgabevorrichtung 7 angeordnet, die mit dem Gantry 3 um 360° um den Patienten herum bewegt werden kann, um eine Bestrahlung aus allen Richtungen zu gewährleisten. Ebenso ist eine Strahlabgabevorrichtung 7 für andere geladene Teilchen im Rahmen der Erfindung einsetzbar. Die Protonenstrahlabgabevorrichtung 7 weist an ihrem vorderen Ende eine Nozzle 9 auf, die zur Applikation des Strahls auf den Patienten dient. Bei der Applikation weist der Strahl eine gemäß dem Bestrahlungsplan vorbestimmte Intensität auf und wird durch über einen Computer gesteuerte Magnete (nicht gezeigt) derart abgelenkt, daß ein exaktes Abscannen des Tumorgewebes mit dem Teilchenstrahl ermöglicht wird.

Die Vorrichtung zur Positionsverifizierung besteht im dargestellten Beispielsfall aus zwei Röntgenstrahlenquellen 11, die in Richtung zur Patientenliege 5 hin ausgerichtet sind, und zwei auf der anderen Seite der Vorrichtung zur Patientenpositionierung 5 angeordneten Röntgenstrahlendetektoren 13, die geeignet sind, die durch die Röntgenstrahlen übermittelten Informationen in Bildinformationen umzuwandeln. Die Röntgenstrahlungsquellen 11 weisen vorzugsweise einen kleinen Brennfleck auf und sind kurz gepulst, während als Röntgenstrahlendetektor 13 Flatpanels aus amorphem Silizium eingesetzt werden. Diese Panels besitzen im dargestellten Ausführungsbeispiel eine Detektionsfläche von ca. 30 x 40 cm. Die Röntgenstrahlungsquellen 11 sind fest im Gantryring 3 installiert, so daß sie bei jeder Bewegung des Gantrys automatisch mitgedreht werden. Auch die Röntgenstrahlendetektoren 13 sind über eine geeignete Mechanik mit dem Gehäuse der Protonenstrahlabgabevorrichtung 7 verbunden.

Es sind viele Anordnungen der Röntgenstrahlungsquellen 11 und Röntgenstrahlendetektoren 13 denkbar, welche die erfindungsgemäßen Vorteile liefern, wichtig ist jedoch eine Anordnung der Röntgenstrahlendetektoren 13 retrograd zur Strahlrichtung des Protonenstrahls. Der Winkel zwischen den beiden Röntgenstrahlendetektoren 13 sollte außerdem nicht zu klein sein, um noch einen ausreichenden Winkelbereich aufnehmen zu können, was die Sicherheit bei der Positionskontrolle erhöht, weil dann in jeder Position der Protonenstrahlabgabevorrichtung 7 eine gesicherte Aussage über die Lage des Patienten getroffen werden kann. Hierzu sollte der Winkel zwischen den beiden Röntgenstrahlendetektoren 13 vorzugsweise nicht kleiner als 60° sein, wobei ein Winkel von über 120° zwar möglich ist, aber aufgrund der auftretenden Streustrahlung während der Protonenbestrahlung nicht für einen gleichzeitigen Einsatz von Bestrahlungsapparat und Positionsverifizierungsvorrichtung geeignet ist, da sich die Röntgenstrahlendetektoren 13 dann nicht mehr in der Zone niedrigster Streustrahlungsintensität befinden würden. Besonders bevorzugt ist eine Anordnung der Röntgenstrahlendetektoren unter einem Winkel von ca. 70°, wobei bis zu einem Bereich von ca. 90° ideale Ergebnisse erzielt werden können.

Der Einsatz der erfindungsgemäßen Vorrichtung zur überwachten Tumorbestrahlung läuft nun folgendermaßen ab: Zunächst wird der Patient auf den für ihn vorgefertigten Tisch im Gantry gelegt. Die aus den Daten der vorangegangenen CT-Untersuchungen ermittelten kartesianischen Summationsbilder, die zur Bestimmung der Bildachsen der Röntgenstrahlenquellen errechnet und dabei perspektivisch korrigiert wurden, liefern ein Referenzbild ("DRR": digitally reconstructed radiograph), aus dem ersichtlich ist, wie bestimmte Fixpunkte des knöchernen Skeletts angeordnet sein müssen, wenn der Patient in der richtigen Position liegt. Nunmehr wird über zumindest eine der Röntgenstrahlungsquellen 11 Röntgenstrahlung emittiert, die durch den auf der Liege 5 befindlichen Patienten teilweise hindurchtritt und von mindestens einem Röntgenstrahlendetektor 13 detektiert wird. Dieser wandelt die erhaltenen Informationen in Bildinformationen um, welche hierauf entweder manuell oder durch eine geeignete Computersoftware mit dem berechneten Bild verglichen werden können. Für etwaige Korrekturen kann entweder der Patient selbst bewegt werden, oder der Patiententisch 5 wird im Raum gedreht, um geringe Unregelmäßigkeiten auszugleichen. Auch eine Bewegung der Protonenstrahlabgabevorrichtung 7 ist möglich.

Wenn sich der Patient in der idealen Position befindet, wird dieser durch die Protonenstrahlabgabevorrichtung 7 bestrahlt, wobei der Tumor in einem Rasterscan-Verfahren abgetastet wird. Die Protonen geben ihre Energie innerhalb des Tumorgewebes ab und zerstören dieses hierdurch. In kurzen Bestrahlungspausen bzw. selbst während der Bestrahlung können weitere Röntgenstrahlimpulse aus den Röntgenstrahlungsquellen 11 emittiert werden, so daß die erfindungsgemäße Vorrichtung auch zur Online-Kontrolle verwendet werden kann, wenn es mit einem geeigneten Computersystem verbunden ist. Dann ist es etwa möglich, das System zu einer quasi kontinuierlichen Dauerüberwachung einzusetzen, die den Bestrahlungsvorgang automatisch abbricht, wenn Unregelmäßigkeiten auftreten. Da die Röntgenstrahlendetektoren 13 retrograd zur Protonenstrahlrichtung angeordnet sind, werden die von den Röntgenstrahlungsdetektoren 13 gelieferten Bilder kann durch von der Protonenbestrahlung herrührende Streustrahlung gestört.

Mit diesem System besteht also die Möglichkeit, Patienten einer Strahlentherapie zu unterziehen und gleichzeitig deren Positionsstatus zu überwachen, unabhängig von der Strahlrichtung der Protonen und des zu bestrahlenden Tumors, wodurch die Bestrahlungssicherheit wesentlich verbessert wird.

Es ist auch denkbar, drei oder mehr Röntgenstrahlungsquellen und -detektoren einzusetzen, um eine möglichst dreidimensionale Bildaufnahme sicherzustellen, wenn es erforderlich sein sollte.

## Patentansprüche

1. Vorrichtung zur überwachten Tumorbestrahlung mit einer Vorrichtung (7) zur Abgabe eines Behandlungsstrahls geladener Teilchen, wobei die Vorrichtung zur überwachten Tumorbestrahlung als drehbares Gantry (3) ausgebildet ist und eine Vorrichtung (5) zur Patientenpositionierung aufweist, die geeignet ist, den Patienten während der Bestrahlung möglichst immobilisiert zu halten, sowie eine Vorrichtung (5) zur Positionsverifizierung des Patienten vor bzw. während der Bestrahlung mit zwei Röntgenstrahlungsquellen (11) und zwei diesen gegenüberliegenden Röntgenstrahlendetektonen (13), die in der jeweiligen Röntgenstrahlachse der zugehörigen Röntgenstrahlungsquelle (11) angeordnet sind wobei die Röntgenstrahlungsquellen (11) sowie die Röntgenstrahlendetektoren (13) fest mit dem Ring des Gantrys (3) verbunden und somit drehbar sind, wobei die Vorrichtung (5) zur Patientenpositionierung jeweils zwischen Röntgenstrahlungsquelle (11) und Röntgenstrahlendetektor (13) positioniert ist, und wobei die Röntgenstrahlendetektoren (13) im Innenraum des Gantryringes an einer radialen Position mit einem Radius, der kleiner ist als der Innenradius des Gantryringes, angeordnet sind,
wobei
die beiden Röntgenstrahlendetektoren (13) in einem Winkel von zwischen 30° und 60° seitlich von der Strahlabgabevorrichtung (7) angeordnet sind, an dem Gehäuse der Strahlabgabevorrichtung (7) befestigt sind und einen Winkel von zwischen 60° und 120° einschließen, dass die Vorrichtung (7) zur Abgabe eines Behandlungsstrahls geeignet ist, den Tumor in einem Rasterscan-Verfahren abzutasten, und dass die Röntgenstrahlendetektoren (13) als Flatpanels aus amorphem Silizium ausgebildet sind.

2. Vorrichtung nach Anspruch 1, **dadurch gekennzeichnet, dass** die beiden Röntgenstrahlendetektoren (13) in einem Winkel von zwischen 35° und 45° seitlich von der Strahlabgabevorrichtung (7) angeordnet sind.

3. Vorrichtung nach Anspruch 1 oder 2, **dadurch gekennzeichnet, dass** die Röntgenstrahlendetektoren (13) im selben Abstand auf beiden Seiten der Strahlabgabevorrichtung (7) angeordnet sind.

4. Vorrichtung nach einem der vorangehenden Ansprüche, **dadurch gekennzeichnet, dass** die Röntgenstrahlendetektoren (13) einen Winkel von zwischen 70° und 90° einschließen.

5. Vorrichtung nach einem der vorangehenden Ansprüche, **dadurch gekennzeichnet, dass** die Vorrichtung (5) zur Patientenpositionierung bewegbar ausgebildet ist.

## Claims

1. Device for monitored tumour irradiation, having a device (7) for emission of a treatment beam of charged particles, the device for monitored tumour irradiation being configured as a rotatable gantry (3) and having a device (5) for patient positioning which is suitable for keeping the patient as immobilised as possible during irradiation, and a device (5) for verifying the position of the patient before or during irradiation, which has two X-radiation sources (11) and two X-ray detectors (13) which are situated opposite said X-radiation sources (11) and are disposed in the respective X-ray axis of the associated X-radiation source (11), the X-radiation sources (11) and the X-ray detectors (13) being connected in a fixed manner to the ring of the gantry (3) and hence being rotatable, the device (5) for patient positioning being positioned respectively between X-radiation source (11) and X-ray detector (13), and the X-ray detectors (13) being disposed at a radial position with a radius which is smaller than the inner radius of the gantry ring,
wherein
both X-ray detectors (13) are disposed at an angle of between 30° and 60° from the beam-emission device (7), are fixed on the housing of the beam-emission device (7) and enclose an angle of between 60° and 120°, the device (7) for emission of a treatment beam is suitable for scanning the tumour in a raster scan method and the X-ray detectors (13) are configured as flat panels made of amorphous silicon.

2. Device according to claim 1, **characterised in that** both X-ray detectors (13) are disposed at an angle of between 35° and 45° laterally of the beam-emission device (7).

3. Device according to claim 1 or 2, **characterised in that** the X-ray detectors (13) are disposed at the same spacing on both sides of the beam-emission device (7).

4. Device according to one of the preceding claims, **characterised in that** the X-ray detectors (13) enclose an angle of between 70° and 90°.

5. Device according to one of the preceding claims, **characterised in that** the device (5) for patient positioning is configured moveably.

## Revendications

1. Dispositif d'irradiation de tumeur contrôlée comprenant un dispositif (7) pour l'émission d'un faisceau de traitement de particules chargées, le dispositif d'irradiation de tumeur contrôlée étant réalisé sous forme de portique rotatif (3), et un dispositif (5) pour le positionnement du patient adapté à une immobilisation maximale du patient pendant l'irradiation, ainsi qu'un dispositif (5) pour la vérification de la position du patient avant ou pendant l'irradiation, qui présente deux sources de rayonnement X (11) et deux détecteurs de rayons X (13) en vis-à-vis de ces dernières, qui sont disposés chacun dans l'axe du faisceau de la source de rayonnement X (11) correspondante, les sources de rayonnement X (11) ainsi que les détecteurs de rayons X (13) étant assemblés fixement avec l'anneau du portique (3) et pouvant ainsi tourner, le dispositif (5) pour le positionnement du patient étant positionné à chaque fois entre la source de rayonnement X (11) et le détecteur de rayons X (13), et les détecteurs de rayons X (13) étant disposés dans l'espace intérieur de l'anneau de portique en une position radiale, dont le rayon est plus petit que le rayon intérieur de l'anneau de portique,
dispositif dans lequel
les deux détecteurs de rayons X (13) sont montés sous un angle de 30° à 60° sur les côtés du dispositif d'émission de faisceau (7), sont fixés sur le carter du dispositif d'émission de faisceau (7) et forment entre eux un angle de 60° à 120°, le dispositif (7) est adapté à l'émission d'un faisceau de traitement pour balayer la tumeur dans un procédé de scanning, et les détecteurs de rayons X (13) sont réalisés sous forme de panneaux plats en silicium amorphe.

2. Dispositif suivant la revendication 1, **caractérisé en ce que** les deux détecteurs de rayons X (13) sont disposés sous un angle de 35° à 45° sur les côtés du dispositif d'émission de faisceau (7).

3. Dispositif suivant l'une des revendications 1 et 2, **caractérisé en ce que** les détecteurs de rayons X (13) sont disposés à la même distance sur les deux côtés du dispositif d'émission de faisceau (7).

4. Dispositif suivant l'une des revendications précédentes, **caractérisé en ce que** les détecteurs de rayons X (13) forment entre eux un angle de 70° à 90°.

5. Dispositif suivant l'une des revendications précédentes, **caractérisé en ce que** le dispositif (5) pour le positionnement du patient est réalisé de manière mobile.
